# FASCICULE DE BREVET EUROPEEN

(11) **EP 3 323 457 B1**
(45) Date de publication et mention de la délivrance du brevet: **26.08.2020**
(21) Numéro de dépôt: 17001688.5
(22) Date de dépôt: 06.04.2016
(51) Int. Cl.: A61M 16/00, A61H 31/00, G09B 23/28

(54) **APPAREIL DE VENTILATION ARTIFICIELLE AVEC MODES VENTILATOIRES ADAPTÉS AU MASSAGE CARDIAQUE**
GERÄT ZUR KÜNSTLICHEN BEATMUNG MIT AN DIE HERZMASSAGE ANGEPASSTEN BEATMUNGSMODI
MECHANICAL VENTILATION APPARATUS WITH VENTILATION MODES SUITABLE FOR CARDIAC MASSAGE

(30) Priorité: 28.04.2015 FR 1553809
(43) Date de publication de la demande: 23.05.2018
(62) Demande divisionnaire de: 16729606.0
(73) Titulaire: Air Liquide Medical Systems, 92160 Antony (FR)
(72) Inventeur: DERMEL, Marius, 75003 Paris (FR); JACQUOT, Eric, 92160 Antony (FR); PENNORS, Thomas, 92160 Antony (FR); RICHARD, Jean-Christophe, 92160 Antony (FR); RIGOLLOT, Marceau, 92120 Montrouge (FR)
(74) Mandataire: Pittis, Olivier

(56) Documents cités:
- EP-A1- 2 198 823
- EP-A2- 2 343 097
- WO-A1-00/20061
- DE-A1-102012 024 672
- FR-A1- 3 000 893

## Description

L'invention concerne un appareil de ventilation artificielle équipé d'une micro-soufflante générant un flux gazeux, en particulier un flux d'air, permettant d'apporter une assistance ventilatoire, en particulier ventilation et monitorage, à un secouriste ou tout autre personnel soignant, tel un médecin du SAMU, un pompier, un infirmier ou analogue, lorsque celui-ci pratique un massage cardiaque sur une personne en arrêt cardiaque.

Il est usuel d'utiliser un appareil de ventilation artificielle, encore appelé appareil d'assistance respiratoire ou ventilatoire, ou plus simplement ventilateur médical, pour apporter une assistance respiratoire, c'est-à-dire une ventilation artificielle, à une personne ayant des difficultés ou une incapacité à respirer seule.

En particulier, il est indispensable de ventiler une personne pendant un arrêt cardiaque afin de continuer à apporter de l'oxygène à son cerveau et au reste du corps, alors que son cœur est arrêté.

Cependant, opérer une ventilation sur une personne en arrêt cardiaque, alors qu'un massage cardiaque est pratiqué sur cette personne, est problématique car cette ventilation ne doit pas interrompre les compressions thoraciques (CT) et/ou ne doit pas être excessive pour ne pas avoir d'effets hémodynamiques négatifs.

Or, les ventilateurs conventionnels ne sont pas adaptés à cette situation, en particulier ils émettent des alarmes et/ou dysfonctionnent pendant les compressions thoraciques.

Dès lors, en pratique, les personnels soignants délivrent les insufflations, via un ventilateur ou un ballon auto-remplisseur à valve unidirectionnelle (BAVU) en interrompant parfois les compressions thoraciques.

La continuité et la régularité du massage cardiaque est un facteur d'efficacité sur le pronostic du patient en arrêt cardiaque. Il n'est donc nullement conseillé de stopper un massage cardiaque, même pour opérer des insufflations de gaz respiratoire au patient.

En outre, les insufflations délivrées manuellement, via un BAVU, ou par un ventilateur conventionnel sont le plus souvent trop agressives, notamment du fait de grands volumes de gaz administrés, entraînant un effet direct délétère et reconnu sur l'efficacité des compressions thoraciques.

Pour cette raison, des appareils évitant les insufflations classiques, notamment des ventilateurs à pression positive continue de type CPAP (Continuous Positive Airway Pressure) ont été proposés en vue d'être utilisés en cas d'arrêt cardiaque. Cependant, ces appareils ne sont pas idéaux car ils ne permettent pas d'assurer une ventilation suffisante de la personne en arrêt cardiaque et l'arrêt des compressions engendre un arrêt de toute ventilation, ce qui n'est pas souhaitable.

Par ailleurs, la plupart des appareils capables de délivrer une ventilation mécanique pendant un massage cardiaque ne permettent pas d'assurer cette ventilation de façon autonome/automatique, alors que cette situation clinique complexe nécessite une grande simplicité d'utilisation. En effet, ces appareils requièrent parfois une intervention humaine lors de chaque insufflation, ce qui complique l'intervention du personnel soignant et conduit parfois à une administration de gaz à une pression et/ou à un volume mal maîtrisés. Or, si pendant une insufflation de gaz respiratoire, la pression et le volume générés ne sont pas correctement maitrisés, il peut se produire des phénomènes néfastes au patient, comme une insufflation gastrique indésirable. Dès lors, avec les appareils classiques, il n'est pas possible de délivrer de façon sûre et simple, une ventilation protectrice qui ne dépasse pas le volume de gaz recommandé.

En outre, au cours du massage cardiaque les compressions de la cage thoracique génèrent une ventilation pulmonaire substantielle mais insuffisante, laquelle doit être complétée par une ventilation mécanique. Quand les compressions thoraciques sont interrompues, après reprise d'activité circulatoire spontanée du patient ou après un choc électrique par exemple, la ventilation diminue brutalement, alors que les besoins en oxygène du malade augmentent. Un complément de ventilation est alors nécessaire imposant des manipulations complexes pour changer de mode de ventilation afin de pouvoir palier ce manque d'oxygène.

Ensuite, une fois ce changement de ventilation opéré et le patient ventilé de façon adaptée, un nouvel arrêt cardiaque peut survenir. Dans ce cas, les compressions thoraciques reprennent et il est alors à nouveau nécessaire de modifier manuellement les réglages du dispositif de ventilation pour se repositionner dans le mode de ventilation le plus adapté pour opérer une réanimation cardio-pulmonaire (RCP).

Un patient peut connaître ainsi plusieurs arrêts pendant sa prise en charge par l'équipe médicale, et délivrer une ventilation optimale nécessite, lors de la survenue de chacun de ces arrêts, une intervention de l'équipe médicale pour adapter l'appareil.

Or, en situation d'urgence, le temps consacré à ces adaptations est au détriment d'opérations plus importantes. Faute de temps, ces adaptations peuvent être oubliées ou négligées, ce qui n'est pas acceptable pour des questions évidentes de sécurité.

De façon générale, la plupart des appareils de ventilation mécanique utilisés actuellement, pendant un massage cardiaque, ne disposent pas d'un mode spécifique adapté à cette situation. Ils sont simplement équipés de déclencheurs inspiratoires qui s'activent souvent à tort et provoquent des auto-déclenchements et des cycles néfastes au débit sanguin cardiaque provoqué par les CT. De plus, certains n'offrent pas la possibilité de régler une pression expiratoire positive (PEP) pourtant indispensable.

Enfin, de nombreuses alarmes sonores et/ou visuelles équipant ces appareils classiques s'activent également à tort, par exemple des alarmes de pression, de volume ou de fréquence car elles sont développées pour des applications « classiques » et que les déclencheurs et les alarmes utilisent les signaux de pression et de débit mesurés par la machine, qui sont, quant à eux, très perturbés par le massage cardiaque.

On connait de FR-A-300893, un appareil d'assistance respiratoire mettant en œuvre un procédé de commande comprenant une mesure d'un paramètre représentatif du flux gazeux et l'utilisation de ce paramètre pour en déduire une information relative à un massage cardiaque opéré sur un patient en arrêt cardiaque et sélectionner automatiquement un mode ventilatoire donné parmi plusieurs modes ventilatoires mémorisés. Toutefois, ce document ne donne pas de détail quant aux modes ventilatoires à appliquer.

Des appareils ou procédés analogues sont décrits par les documents EP-A-2198823, WO-A-00/20061 et EP-A-2343097.

Au vu de cela, le problème qui se pose est dès lors de proposer un appareil de ventilation artificielle, c'est-à-dire un appareil d'assistance respiratoire, encore appelé ventilateur médical, et un procédé de commande ou de pilotage d'un tel appareil de ventilation artificielle, permettant de résoudre tout ou partie des problèmes et inconvénients susmentionnés, en apportant une réponse adaptée en termes de mode ventilatoire en fonction de l'absence ou de la présence de contractions thoraciques, c'est-à-dire de massage cardiaque pratiqué sur un patient en arrêt cardio-pulmonaire.

De préférence, l'appareil et le procédé doivent permettre de détecter la réalisation d'un massage cardiaque sur le patient en arrêt cardiaque sans intervention humaine, c'est-à-dire de façon automatique.

De plus, l'appareil et le procédé doivent permettre d'apporter une ventilation mécanique adaptée à la présence ou à l'absence du massage cardiaque, en prenant en compte les phases de compression et celles de décompression thoraciques, dans le but d'améliorer l'efficacité de la ventilation apportée au patient et ce, sans déclencher d'alarmes intempestives.

En d'autres termes, le procédé de l'invention doit permettre de couvrir toutes les étapes de la réanimation cardio-pulmonaire (RCP) sans interruption, en particulier des phases cycliques de compression/décompression thoracique, et donc au final d'améliorer la ventilation en évitant les interruptions de ventilation et/ou toute ventilation excessive.

La solution de l'invention concerne alors un appareil d'assistance respiratoire, c'est-à-dire un ventilateur médical, selon la revendication 1.

Selon le cas, l'appareil ou ventilateur médical peut comprendre l'une ou plusieurs des caractéristiques techniques suivantes :
- selon un premier mode de réalisation, les valeurs de première et deuxième pression basse (PB1, PB2) sont égales, c'est-à-dire PB1 = PB2. Dans ce cas, la pression basse n'est pas modifiée et est maintenue constante à une valeur fixe donnée PB et on a dès lors PB1=PB2=PB.
- selon un deuxième mode de réalisation, les valeurs de première et deuxième pression basse (PB1, PB2) sont différentes, c'est-à-dire que PB1 < PB2. Dans ce cas, la pression basse est modifiée en fonction du mode ventilatoire sélectionné.
- les moyens de sélection de mode ventilatoire sont conçus pour opérer un basculement du premier mode ventilatoire vers le deuxième mode ventilatoire, ou inversement, de manière à modifier en outre la pression basse (PB1, PB2) en réponse à une détection d'au moins une information relative à une absence ou un arrêt de contractions thoraciques ou, inversement, une présence de contractions thoraciques par les moyens de traitement de signal.
- les moyens de traitement de signal permettent de détecter les tentatives de respirations spontanées du patient, par analyse des perturbations présentes sur les signaux de pression et/ou de débit. Une fois détectées, le ventilateur médical délivre un cycle de ventilation pour y répondre, entre une pression basse (PB1, PB2) et une pression haute (PH1, PH2). Ce mécanisme de synchronisation, entre le besoin patient et la ventilation délivrée, est usuellement appelé Trigger. Ce mécanise de synchronisation est actif quand le deuxième mode ventilatoire est appliqué.
- à l'inverse, ce mécanisme de synchronisation, Trigger, doit être désactivé lorsque le premier mode ventilatoire est activé. La présence des compressions thoraciques empêche la bonne détection des éventuelles tentatives de respirations spontanées du patient, de plus en situation d'arrêt cardiaque, ce besoin de respiration spontanée est physiologiquement inhibé. Par conséquent, ce mécanisme de synchronisation, Trigger, doit être désactivé lorsque le premier mode ventilatoire est activé.
- les moyens de traitement de signal sont configurés pour opérer en continu et assurer une détection de contractions thoraciques correspondant à une information de réalisation ou de une non-réalisation de massage cardiaque, et à fournir ladite information aux moyens de sélection de mode ventilatoire de manière à ce que lesdits moyens de sélection de mode ventilatoire opèrent un basculement, c'est-à-dire le passage, d'un mode ventilatoire donné à un autre mode ventilatoire donné en fonction de la présence ou de l'absence de contractions thoraciques, c'est-à-dire d'un massage thoracique.
- les moyens de sélection de mode ventilatoire sont conçus pour opérer un basculement du premier mode ventilatoire vers le deuxième mode ventilatoire en réponse à une détection d'au moins une information relative à une absence ou un arrêt de contractions thoraciques par les moyens de traitement de signal.
- à l'inverse, les moyens de sélection de mode ventilatoire sont conçus pour opérer un basculement du second mode ventilatoire vers le premier mode ventilatoire en réponse à une détection d'au moins une information relative à une existence ou présence de contractions thoraciques par les moyens de traitement de signal.
- optionnellement, les moyens de mémorisation de modes ventilatoires sont configurés pour mémoriser un premier mode ventilatoire comprenant en outre une valeur de première concentration en oxygène (FiO₂-1) donnée et un second mode ventilatoire comprenant une valeur de deuxième concentration en oxygène (FiO₂-2) donnée et les moyens de sélection de mode ventilatoire sont conçus pour opérer un basculement du premier mode ventilatoire vers le deuxième mode ventilatoire de manière à modifier en outre la concentration en oxygène (FiO₂-1, FiO₂-2) en réponse à une détection d'au moins une information relative à une absence ou un arrêt de contractions thoraciques par les moyens de traitement de signal.
- les moyens de mémorisation de modes ventilatoires sont configurés pour mémoriser un premier mode ventilatoire ne comprenant pas de moyen de synchronisation, entre les cycles de ventilation et les besoins patient, et un second mode ventilatoire comprenant un ou des moyens de synchronisation, entre les cycles de ventilation et les besoins patient, et les moyens de sélection de mode ventilatoire sont conçus pour opérer un basculement du premier mode ventilatoire vers le deuxième mode ventilatoire de manière à activer le ou les moyens de synchronisation entre les cycles de ventilation et les besoins patient, en réponse à une détection d'au moins une information relative à une absence ou un arrêt de contractions thoraciques par les moyens de traitement de signal.
- les moyens de mémorisation de modes ventilatoires sont configurés pour mémoriser un premier mode ventilatoire comprenant des valeurs de première pression basse (PB1) comprise entre 0 et 20 cm d'eau, de première pression haute (PH1) comprise entre 10 et 60 cm d'eau, avec PH1>PB1, et de première fréquence (F1) comprise entre 5 et 40 c/min.
- les moyens de mémorisation de modes ventilatoires sont configurés pour mémoriser un second mode ventilatoire comprenant des valeurs de deuxième pression basse (PB2) comprise entre 0 et 20 cm d'eau, de deuxième pression haute (PH2) comprise entre 10 et 60 cm d'eau, avec PH2>PB2, PB2≥PB1 et PH2≥PH1, c'est-à-dire que selon le cas, PB1=PB2=PB ou PB1<PB2, et de deuxième fréquence (F2) comprise entre 5 et 40 c/min, avec F2>F1.
- les moyens de sélection de mode ventilatoire permettant de sélectionner et d'appliquer automatiquement le premier ou le deuxième mode ventilatoire mémorisé, en fonction de ladite au moins une information fournie par les moyens de traitement de signal. Dans ce cas, le premier ou le deuxième mode ventilatoire est sélectionné automatiquement par l'appareil en fonction de l'information (ou des informations) fournie par les moyens de traitement de signal. Il n'y a donc pas d'intervention humaine dans ce mode de réalisation.
- selon un autre mode de réalisation, les moyens de sélection de mode ventilatoire permettant de sélectionner et d'appliquer le premier ou le deuxième mode ventilatoire mémorisé, en réponse à l'activation par l'utilisateur d'un sélecteur de mode ventilatoire, par exemple un (ou plusieurs) bouton, une touche ou analogue, notamment une (ou plusieurs) touche située sur un écran tactile, c'est-à-dire sur une interface homme-machine (IHM). Dans ce cas, c'est l'activation par l'utilisateur du sélecteur de mode ventilatoire qui va autoriser une sélection et une application du premier ou du deuxième mode ventilatoire mémorisé par les moyens de sélection de mode ventilatoire. A titre d'exemple, l'appareil peut proposer un mode ventilatoire à l'utilisateur et ce dernier, peut le valider ou non par action sur une touche ou analogue.
- les moyens de sélection de mode ventilatoire sont configurés pour opérer selon le premier mode ventilatoire en cas de détection de présence de contractions thoraciques.
- les moyens de mesure comprennent au moins un capteur.
- les moyens de traitement de signal comprennent au moins une carte électronique.
- les moyens de traitement de signal comprennent au moins un microprocesseur.
- les moyens de traitement de signal comprennent au moins un (micro)contrôleur mettant en œuvre un ou plusieurs algorithmes.
- les moyens de sélection de mode ventilatoire comprennent des moyens de réglage ou de sélection, par exemple des boutons poussoirs ou rotatifs, des curseurs, des touches d'activation ou de sélection, ou analogue, permettant au personnel soignant d'interagir, c'est-à-dire de commander, avec le ventilateur et de modifier les paramètres de la ventilation délivrée.
- les moyens de mémorisation de modes ventilatoires comprennent au moins une mémoire de stockage de données, en particulier une mémoire flash ou analogue.
- une micro-soufflante motorisée délivrant un gaz respiratoire, typiquement de l'air ou de l'air enrichi en oxygène.
- il comprend un circuit de gaz apte à véhiculer le gaz respiratoire délivré par la micro-soufflante et destiné à être administré à un patient en arrêt cardiaque.
- la micro-soufflante est pilotée/commandée par des moyens de pilotage.
- la micro-soufflante motorisée, encore appelée turbine, est équipée d'un moteur électrique.
- au moins une partie du circuit de gaz, les moyens de pilotage, les moyens de traitement de signal sont situés dans une carcasse rigide, c'est-à-dire une enveloppe externe formant la coque ou le capotage de l'appareil.
- le circuit de gaz comprend une portion interne agencée dans la carcasse rigide et une portion externe située hors de la carcasse rigide.
- la portion interne du circuit de gaz comprend un conduit de gaz.
- la portion interne du circuit de gaz est en communication fluidique avec une source de gaz de manière à être alimenté avec du gaz délivré par ladite de gaz.
- les moyens de mesure sont agencés sur la portion externe du circuit de gaz située hors de la carcasse rigide de manière à opérer des mesures au sein de ladite portion externe.
- les moyens de mesure sont agencés sur la portion interne du circuit de gaz située dans la carcasse rigide de manière à opérer des mesures au sein de ladite portion interne.
- il comprend plusieurs moyens de mesure dont certains sont agencés sur la portion interne du circuit de gaz et d'autres sur la portion externe du circuit de gaz.
- les moyens de mesure comprennent des capteurs.
- il comprend en outre une interface homme-machine (IHM) apte à, c'est-à-dire conçue pour, afficher des informations incluant au moins une information relative à la réalisation d'un massage cardiaque sur le patient en arrêt cardiaque.
- l'interface homme-machine comprend un écran d'affichage et/ou de visualisation d'informations, tel un écran digital, en particulier un écran tactile.
- la portion externe du circuit de gaz est en communication fluidique avec une interface respiratoire, en particulier un masque respiratoire.
- la portion externe du circuit de gaz comprend un tube ou conduit flexible.
- la source de gaz est une source d'air (env. 21 vol. % d'O₂) ou d'air enrichi en oxygène (>21 vol.% d'O₂ environ, typiquement >50% en vol).- la carcasse comprend au moins une poignée de portage facilitant le transport de l'appareil par un utilisateur.
- la carcasse comprend au moins un dispositif d'accrochage permettant l'accroche de l'appareil de ventilation à un support, par exemple une tringle au sein d'un véhicule d'urgence ou un barreau de lit ou de brancard.
- il comprend des moyens d'alimentation en courant électrique, par exemple une (ou plusieurs) batterie ou analogue, ou un (ou plusieurs) câble et une (ou plusieurs) prise de raccordement au secteur électrique.
- il comporte en outre des moyens de réglage et de sélection, tel que bouton-poussoir, touche d'activation, curseur ou analogue, permettant au personnel soignant d'intervenir au niveau du ventilateur, par exemple d'indiquer au ventilateur, la réalisation d'un massage cardiaque, de lui confirmer une détection d'un massage cardiaque, de lui indiquer le type d'interface respiratoire utilisée (masque, sonde d'intubation...), de modifier un ou des paramètres de ventilation mécanique proposés automatiquement par le ventilateur, ou tout autre indication.
- il comprend des moyens de pilotage incluant les moyens de traitement de signal.
- il comprend une micro-soufflante en communication fluidique avec la branche inspiratoire du circuit de gaz de manière à l'alimenter en gaz délivré par ladite micro-soufflante et les moyens de pilotage sont configurés pour commander ladite micro-soufflante de manière à ajuster la pression ou la fréquence du gaz délivré, typiquement de l'air éventuellement enrichi en oxygène.
- selon un autre mode de réalisation, il comprend une première vanne pilotée agencée sur ledit circuit de gaz, en particulier sur la branche inspiratoire dudit circuit, permettant de réguler la circulation de gaz alimentant ledit circuit, ledit gaz provenant d'une canalisation extérieure raccordée à l'appareil, et les moyens de pilotage sont configurés pour commander ladite première vanne pilotée de manière à ajuster la pression ou la fréquence du gaz délivré, typiquement de l'air éventuellement enrichi en oxygène.

En outre un procédé de commande, c'est-à-dire de pilotage, d'un appareil d'assistance respiratoire délivrant un flux de gaz, en particulier un flux d'air, P est décrit ici; ce procédé de commande, en particulier de l'appareil selon l'invention décrit ci-avant, comprenant les étapes de :
a) mesurer au moins un paramètre représentatif dudit flux de gaz,
b) convertir ledit au moins un paramètre représentatif dudit flux de gaz en au moins un signal représentatif dudit flux de gaz,
c) traiter ledit au moins un signal représentatif du flux de gaz pour en déduire au moins une information relative à un massage cardiaque opéré sur un patient en arrêt cardiaque,
d) sélectionner un mode ventilatoire donné parmi plusieurs modes ventilatoires mémorisés, et
e) commander l'appareil d'assistance respiratoire en appliquant le mode ventilatoire sélectionné à l'étape d).

Selon le procédé de commande :
- on mémorise plusieurs modes ventilatoires comprenant :
   i) un premier mode ventilatoire, dit de ventilation avec massages cardiaques, comprenant des valeurs de première pression basse (PB1), de première pression haute (PH1), avec PH1>PB1 et de première fréquence (F1) données, et optionnellement de première concentration en oxygène (FiO₂-1)) donnée, et
   ii) un second mode ventilatoire, dit de ventilation classique ou normale, c'est-à-dire de ventilation en l'absence de massages cardiaques, comprenant des valeurs de deuxième pression basse (PB2) et de deuxième pression haute (PH2), avec PH2>PB2, et PH2≥PH1, de deuxième fréquence (F2) données, et optionnellement de deuxième concentration en oxygène (FiO₂-2) donnée.
- on opère un basculement du premier mode ventilatoire vers le deuxième mode ventilatoire, ou inversement, de manière à modifier la pression haute (PH1, PH2) et/ou la fréquence (F1, F2) en réponse à une détection d'au moins une information relative à une absence ou un arrêt de contractions thoraciques ou, inversement, une présence de contractions thoraciques par les moyens de traitement de signal.

Selon le cas, le procédé de commande peut comprendre l'une ou plusieurs des caractéristiques techniques suivantes :
- selon un premier mode de réalisation, les valeurs de première et deuxième pression basse (PB1, PB2) sont égales, c'est-à-dire PB1 = PB2. Dans ce cas, la pression basse n'est pas modifiée et est maintenue constante à une valeur fixe donnée, PB.
- selon un deuxième mode de réalisation, les valeurs de première et deuxième pression basse (PB1, PB2) sont différentes, c'est-à-dire que PB1 < PB2. Dans ce cas, la pression basse est modifiée en fonction du mode ventilatoire sélectionné.
- on opère un basculement du premier mode ventilatoire vers le deuxième mode ventilatoire, ou inversement, de manière à modifier en outre la pression basse (PB1, PB2) en réponse à une détection d'au moins une information relative à une absence ou un arrêt de contractions thoraciques ou, inversement, une présence de contractions thoraciques par les moyens de traitement de signal.
- à l'étape c), on opère une détection de contractions thoraciques, de préférence la détection est opérée de manière continue.
- à l'étape c), on opère une détection de contractions thoraciques comprenant une alternance de phases de compression et de relâchement/décompression de la cage thoracique.
- le flux de gaz est délivré par une micro-soufflante équipant l'appareil d'assistance respiratoire ou provient d'une canalisation d'alimentation en gaz raccordée à l'appareil d'assistance respiratoire, en particulier une canalisation d'alimentation en gaz alimentant une prise murale, elle-même connectée fluidiquement à l'appareil d'assistance respiratoire mettant en œuvre le procédé ici décrit.
- le paramètre représentatif du flux de gaz est choisi parmi une pression du gaz, un débit de gaz insufflé au patient, un débit de gaz expiré par le patient et une vitesse de la micro-soufflante.
- lorsqu'à l'étape c), on détecte une absence ou un arrêt de contractions thoraciques, on opère alors un basculement du premier mode ventilatoire vers le deuxième mode ventilatoire de manière à modifier la pression haute, la fréquence et/ou la concentration en oxygène pour prendre en compte l'absence de contractions thoraciques.
- lorsqu'à l'étape c), on détecte au moins une information relative à une présence ou reprise des contractions thoraciques, on opère alors un basculement du second mode ventilatoire vers le premier mode ventilatoire de manière à modifier la pression haute, la fréquence et/ou la concentration en oxygène pour prendre en compte l'existence ou la présence de contractions thoraciques.
- on mémorise plusieurs algorithmes d'alarmes comprenant :
   - un premier ensemble d'algorithmes, dit de surveillance avec massages cardiaques, configurés pour opérer une surveillance d'un ou plusieurs paramètres de ventilation et/ou éléments techniques de l'appareil de ventilation. Ces algorithmes sont spécifiques aux conditions rencontrées pendant un massage cardiaque.
   - un second ensemble d'algorithmes, dit de surveillance classique, configurés pour opérer une surveillance d'un ou plusieurs paramètres de ventilation et/ou éléments techniques de l'appareil. Ces algorithmes sont adaptés à une ventilation classique et peuvent être partagés avec d'autres modes de ventilation présents.
- lorsqu'à l'étape c), on détecte une absence ou un arrêt de contractions thoraciques, on opère alors un basculement du premier ensemble d'algorithmes de surveillance vers le second ensemble d'algorithmes de surveillance de manière à adapter les critères de surveillance à l'absence de massage cardiaque.
- lorsqu'à l'étape c), on détecte au moins une information relative à une présence ou une reprise des contractions thoraciques, on opère alors un basculement du second ensemble d'algorithmes de surveillance vers le premier ensemble d'algorithmes de surveillance de manière à adapter les critères de surveillance à la présence d'un massage cardiaque.
- il comprend une étape d'affichage du mode ventilatoire sélectionné.
- on commande la micro-soufflante motorisée de manière à maintenir une ventilation minute constante en réponse à une mesure d'un volume de gaz échangé entre le patient et l'appareil d'assistance respiratoire qu'un massage cardiaque soit réalisé ou non.
- on commande la micro-soufflante motorisée de manière à délivrer du gaz, alternativement à une pression basse (PB1) donnée et à une pression haute (PH1) donnée, avec PH1>PB1, en cas de détection de massage cardiaque.
- on commande la micro-soufflante motorisée de manière à augmenter la pression haute de ventilation en réponse à une détection d'un arrêt du massage cardiaque ou d'absence de massage cardiaque.
- on commande la micro-soufflante motorisée de manière à diminuer la pression haute de ventilation en réponse à une détection de réalisation d'un massage cardiaque, par exemple lors d'une reprise de massages cardiaques après une phase d'arrêt des massages cardiaques.
- on commande la micro-soufflante motorisée de manière à augmenter la fréquence (F) de ventilation en réponse à une détection d'un arrêt du massage cardiaque.
- on commande la micro-soufflante motorisée de manière à diminuer la fréquence (F) de ventilation en réponse à une détection de réalisation d'un massage cardiaque.
- on commande la micro-soufflante motorisée de manière à augmenter la pression haute (PH2) de ventilation en réponse à une action de l'utilisateur indicative du passage d'un mode de ventilation au masque à un mode de ventilation intubé, c'est-à-dire qu'on augmente la pression du gaz délivré par la micro-soufflante lorsque le patient est intubé.
- l'action de l'utilisateur, typiquement le personnel soignant, indicative du passage d'un mode de ventilation au masque à un mode de ventilation intubé est une activation d'un dispositif de commande, telle une touche, un bouton-poussoir ou analogue, ledit dispositif de commande activable par l'utilisateur étant situé sur l'appareil de ventilation.
- on commande la micro-soufflante motorisée pour délivrer du gaz entre un premier niveau de pression basse (PB1) et un premier niveau de pression haute (PH1), avec PH1>PB1, en réponse à une action de l'utilisateur indicative d'un début de massage cardiaque.
- on commande la micro-soufflante motorisée de manière à augmenter la pression du gaz délivré par la micro-soufflante depuis le premier niveau de pression haute (PH1) jusqu'à atteindre un deuxième niveau de pression haute (PH2), avec PH2>PH1>PB1, en réponse à une action de l'utilisateur indicative de l'arrêt du massage cardiaque. Optionnellement, on augmente en outre la pression basse du gaz d'une valeur PB1 à une valeur PB2 avec PB2>PB1 et/ou on augmente la fréquence de ventilation d'une valeur F1 à une valeur F2 avec F2>F1.
- selon un autre mode de réalisation, on commande la micro-soufflante motorisée pour délivrer du gaz entre un niveau de première pression basse (PB1) et un niveau de première pression haute (PH1), avec PH1>PB1, en réponse à une détection automatique par l'appareil d'un début ou de l'existence d'un massage cardiaque. De façon analogue, on commande la micro-soufflante motorisée de manière à augmenter la pression du gaz délivré par la micro-soufflante depuis le premier niveau de pression haute (PH1) jusqu'à atteindre un deuxième niveau de pression haute (PH2), avec PH2>PH1>PB, en réponse à une détection automatique par l'appareil de l'arrêt ou d'une absence de massage cardiaque. Optionnellement, on augmente aussi automatiquement la pression basse du gaz d'une valeur PB1 à une valeur PB2 avec PB2>PB1 et/ou on augmente la fréquence de ventilation d'une valeur F1 à une valeur F2 avec F2>F1.
- le premier niveau de pression haute (PH1), le deuxième niveau de pression haute (PH2) et le ou les niveaux de pression basse (PB ; PB1, PB2) sont mémorisés.
- le premier niveau de pression haute (PH1), le deuxième niveau de pression haute (PH2) et le ou les niveaux de pression basse (PB ; PB1, PB2) sont ajustables par l'utilisateur.
- d'une façon générale, selon l'invention, on commande la micro-soufflante motorisée de manière à :
   i) augmenter la pression maximale du gaz délivré par la micro-soufflante en réponse à un arrêt ou une absence de massage cardiaque, de préférence on augmente le niveau de pression jusqu'au deuxième niveau de pression haute (PH2), le patient étant alors ventilé entre une pression basse donnée et une pression haute (PH2); et/ou
   ii) diminuer la pression maximale du gaz délivré par la micro-soufflante en réponse à un début ou une existence de massage cardiaque, de préférence on diminue le niveau de pression jusqu'au premier niveau de pression haute (PH1), le patient étant alors ventilé entre une pression basse donnée et une pression haute (PH2).
- à titre indicatif, le niveau de première pression basse (PB1) est compris entre environ 3 et 15 cm d'eau, le niveau de première pression haute (PH1) est compris entre environ 10 et 40 cm d'eau, le deuxième niveau de pression haute (PH2) est compris entre environ 12 et 60 cm d'eau et le niveau de deuxième pression basse (PB2) est compris entre environ 3 et 15 cm d'eau,
- de préférence, la pression basse est constante et (PB 1 = PB2 = PB) est de l'ordre de 5 cm d'eau.
- de préférence, le premier niveau de pression haute (PH1) est de l'ordre de 15 cm d'eau et le deuxième niveau de pression haute (PH2) est de l'ordre de 20 cm d'eau.
- à titre indicatif, la première fréquence de ventilation (F1) est comprise entre environ 5 et 40 c/min et la seconde fréquence de ventilation (F2) est comprise entre environ 10 et 40 c/min.
- de préférence, la première fréquence de ventilation (F1) est de l'ordre de 10 c/min et la seconde fréquence de ventilation (F2) est de l'ordre de 15 c/min.
- on commande la micro-soufflante avec des moyens de pilotage, en particulier une carte électronique, par exemple à microcontrôleur et algorithme(s).

De façon générale, concernant le mode de ventilation spécifique à la réanimation cardio-pulmonaire, celui-ci peut être un mode volumétrique ou barométrique, de préférence associé à une pression basse (PB) minimale de ventilation, par exemple de l'ordre de 5 cm d'H₂O.

Avantageusement, il s'agit d'un mode barométrique qui assure une régulation alternée sur plusieurs niveaux de pression comprenant un unique niveau de pression basse (PB=PB1=PB2) et un ou plusieurs niveaux de pression haute (PH1, PH2), avec PH2>PH1>PB, par exemple une pression basse (PB) de l'ordre de 5 cm d'H₂O, et une première pression haute (PH1) de l'ordre de 15 cm d'H₂O et une deuxième pression haute (PH2) de l'ordre de 20 cm d'H₂O.

Le mode de ventilation spécifique à la réanimation cardio-pulmonaire permet d'assurer la ventilation d'un patient du début à la fin de l'intervention dans un environnement nécessitant peu ou pas d'intervention humaine lors des différentes phases

L'appareil d'assistance respiratoire de l'invention dispose, outre ce mode de ventilation spécifique à la réanimation cardio-pulmonaire, d'autres modes de ventilation conventionnelle comme un ou des modes de ventilation volumétriques (VAC), barométriques (VPC, VSAI, CPAP, Duo-Levels...) et/ou intermittents (VACI, PVACI..).

En d'autres termes, l'appareil conforme à la présente invention permet de couvrir les différentes étapes d'un massage cardiaque, c'est-à-dire les phases avec et sans CT, en permettant une reconnaissance par la mesure de la variation de pression et/ou du débit gazeux dans les voies aériennes du patient, et ensuite une bascule, de préférence automatique, de la ventilation adaptée aux CT à la ventilation adaptée à l'absence de CT, après un retour d'activité circulatoire spontanée (RACS) par exemple.

D'une façon générale, les moyens de traitement de signal, en particulier une carte électronique, par exemple du type à microcontrôleur et algorithme(s), de l'appareil utilisable dans le cadre de l'invention sont aptes à et conçus, par exemple configurés ou programmés, pour sélectionner un mode ventilatoire donné préenregistré, c'est-à-dire mémorisé, qui est spécifique à une réanimation cardio-pulmonaire, parmi plusieurs modes ventilatoires mémorisés en réponse à une détection de la réalisation ou de la non-réalisation d'un massage cardiaque sur le patient, c'est-à-dire à la détection ou non de CT par le ventilateur lui-même.

Le ventilateur permet donc d'opérer une adaptation automatique des réglages du mode ventilatoire et une sélection automatique d'un mode spécifique à mettre en œuvre dans le cadre d'une réanimation cardio-pulmonaire.

En particulier, il permet de maintenir, une ventilation minute constante en l'absence et en présence d'un massage cardiaque et ce, quel que soit le mode de délivrance du massage cardiaque. Ceci se fait par exemple par mesure par les moyens de traitement de signal, du volume de gaz échangé entre le patient et l'appareil d'assistance respiratoire.

La sélection d'un mode ventilatoire spécifique à mettre en œuvre dans le cadre d'une réanimation cardio-pulmonaire engendre une modification des paramètres de la ventilation mécanique apportée au patient, notamment les pressions délivrées (pression basse et pression haute), la fréquence de ventilation, la durée de maintien de la pression basse, la durée de maintien de la pression haute, la pente de montée en pression, le volume délivré à l'insufflation, la nature du gaz délivré...

Il est précisé que, dans le cadre de la présente invention, le terme « moyens » est considéré comme étant strictement équivalent du terme « dispositif ». De là, les termes « moyens de mesure » équivalent à « dispositif de mesure »; les termes « moyens d'affichage » équivalent à « dispositif d'affichage »; les termes « moyens de traitement » équivalent à « dispositif de traitement »; les termes « moyens de stockage de données » équivalent à « dispositif de stockage de données »; etc...

La présente invention va maintenant être décrite plus en détail en références aux Figures annexées parmi lesquelles:
- les Figures 1A et 1B représentent deux modes de réalisation d'un appareil d'assistance respiratoire utilisable pour mettre en œuvre le procédé de commande selon la présente invention;
- la Figure 2 est une représentation des différentes étapes de la prise en charge d'un patient et des adaptations faites par l'appareil d'assistance respiratoire mettant en œuvre le procédé de commande selon la présente invention; et
- les Figures 3A à 3C sont des illustrations détaillées de la Figure 2.

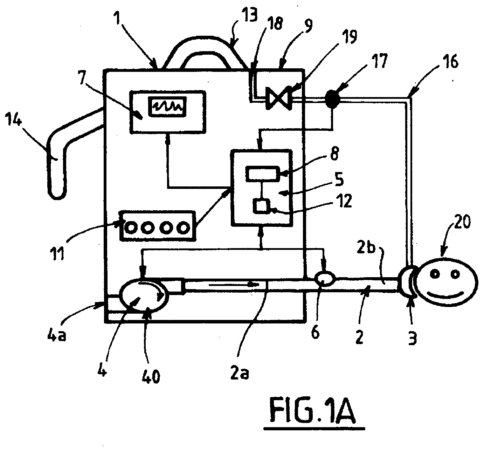

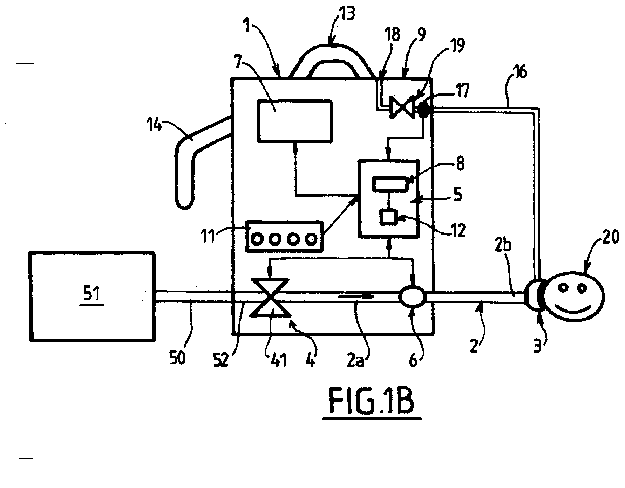

Les Figures 1A et 1B schématisent deux modes de réalisation d'un appareil d'assistance ventilatoire ou ventilateur médical 1 apte à mettre en œuvre le procédé de commande selon la présente invention.

Le ventilateur 1 des Figures 1A et 1B comprend une source de gaz 4 qui est une micro-soufflante motorisée 40 dans le mode de réalisation de la Figure 1A, encore appelée turbine, délivrant un flux de gaz d'assistance respiratoire, typiquement un flux d'air ou d'air enrichi en oxygène, ou une vanne pilotée 41 alimentée en gaz, via un conduit interne 52, lui-même en communication fluidique avec un réservoir de gaz ou une prise murale 51 de fourniture de gaz reliée à un réseau de canalisations de gaz, par l'intermédiaire d'un conduit souple 50 raccordant le réservoir de gaz ou la prise murale 51 au conduit interne 52, dans le mode de réalisation de la Figure 1B.

Dans tous les cas, un circuit ventilatoire 2, 16, encore appelé circuit patient, comprenant un ou plusieurs passages, conduits ou lignes de gaz, permet de relier fluidiquement la source de gaz 4 du ventilateur 1 aux voies aériennes d'un patient 20, par l'intermédiaire d'une interface patient 3, par exemple un masque respiratoire ou une sonde d'intubation.

Le circuit ventilatoire 2, 16 comprend au moins une branche inspiratoire 2 permettant de convoyer le gaz respiratoire au patient 20. Il peut comporter également une branche expiratoire 16 conçue pour recueillir les gaz expirés par le patient 20 qui sont riches en CO₂, comme illustré sur les Figures 1A et 1B. La branche expiratoire 16 comprend un capteur de débit expiratoire 17, par exemple un capteur à fil chaud, relié électriquement aux moyens de traitement de signal et de pilotage 5, 8, ainsi qu'une valve expiratoire 19 pilotée par les moyens de pilotage 5. La branche expiratoire 16 communique, à son extrémité aval, avec l'atmosphère via un orifice de sortie de gaz 18, alors que son extrémité amont est reliée à la branche inspiratoire 2, via une pièce en Y, soit directement à l'interface patient 3.

Des moyens de mesure 6 sont prévus, tel un capteur, lesquels sont aptes à et conçus pour mesurer au moins un paramètre représentatif du flux de gaz choisi parmi la pression du gaz, le débit de gaz insufflé par le respirateur, le débit de gaz expiré par le patient 20 et la vitesse de rotation de la micro-soufflante 40, et à délivrer au moins un signal représentatif dudit au moins un paramètre mesuré.

Par exemple, le paramètre représentatif du flux de gaz est la pression du gaz dans la branche inspiratoire 2 du circuit de gaz ventilatoire 2, 16, et les moyens de mesure 6 comprennent un capteur de pression dont la prise de pression est agencée de manière à permettre une mesure de la pression gazeuse régnant dans ladite branche inspiratoire 2 du circuit ventilatoire 2. Dans le mode de réalisation illustré sur des Figures 1A et 1B, la prise de pression faisant office de moyens de mesure 6, est agencée hors du ventilateur. Toutefois, elle peut également se trouver dans le ventilateur 1, selon un autre mode de réalisation.

Une fois la mesure du (ou des) paramètre(s) représentatif(s) du flux de gaz opérée, ce paramètre mesuré est converti en au moins un signal représentatif du flux de gaz, qui est alors transmis à et analysé par des moyens de traitement de signal 8 pour en déduire au moins une information relative à un massage cardiaque opéré sur un patient en arrêt cardiaque.

Les moyens de traitement de signal 8 font partie des moyens de pilotage 5 du ventilateur 1 et comprennent une ou plusieurs cartes électroniques.

La transmission du (ou des) signal est transmis par les moyens de mesure 6 aux moyens de traitement de signal 8 via une connectique adaptée, c'est-à-dire des liaisons électriques, tel que câbles ou autres.

Ensuite, les moyens de traitement de signal 8 peuvent en déduire ou déterminer :
- qu'un massage cardiaque est en cours de réalisation sur le patient 20 et si la phase en cours est une phase de compression ou une phase de relâchement de la cage thoracique;
- le volume de gaz insufflé par le ventilateur 1 au patient 20, au cours des cycles de ventilation mécanique et pendant les phases de relâchement de la cage de thoracique. Les volumes de gaz insufflé peuvent être cumulés sur une période de temps donnée, par exemple 1 minute. Bien entendu, le cumul peut être opéré sur plus de 1 min ou, à l'inverse, sur moins de 1 min.
- le volume de gaz expiré par le patient 20, au cours des cycles de ventilation mécanique et pendant les phases de relâchement de la cage de thoracique. Là aussi, les volumes de gaz expiré peuvent être cumulés sur une période de temps donnée, par exemple 1 minute ; bien entendu, le cumul peut être opéré sur plus de 1 min ou, à l'inverse, sur moins de 1 min.

En d'autres termes, grâce aux moyens de traitement de signal 8, on opère préférentiellement une détection de contractions thoraciques, de préférence de manière continue, c'est-à-dire une détection d'une alternance de phases de compression et de relâchement/décompression de la cage thoracique.

Les moyens de traitement de signal 8 sont donc aptes à et conçus pour :
i) traiter le signal correspondant au paramètre représentatif du flux de gaz et par exemple y détecter une ou plusieurs variations positives ou négatives supérieures à une ou plusieurs valeurs-seuils représentatives des phases de compression ou de relâchement de la cage thoracique au cours d'un massage cardiaque. Ces valeurs-seuils sont enregistrées dans une mémoire 12 de stockage, par exemple une mémoire flash. Ces valeurs-seuils peuvent être des valeurs numériques, des tableaux de valeurs, des courbes...
ii) intégrer sur le signal correspondant au paramètre représentatif du flux de gaz, le débit de gaz généré par le ventilateur 1 pendant les compressions thoraciques et les cycles générés par la machine.
iii) intégrer par rapport au temps, le signal correspondant au paramètre représentatif du flux de gaz, et le débit de gaz généré par le ventilateur 1 pendant les compressions thoraciques et les cycles générés par le ventilateur 1.
iv) intégrer par rapport au temps, le signal correspondant au paramètre représentatif du flux de gaz, et le débit de gaz expiré par le patient 20 pendant les compressions thoraciques et les cycles générés par le ventilateur 1.

Pour ce faire, les moyens de traitement de signal 8 comprennent de préférence un microprocesseur programmé notamment avec un (ou plusieurs) algorithme(s) de traitement, comme détaillé ci-après.

Ensuite, en fonction de l'information de massage cardiaque déduite, le ventilateur 1 opère une sélection automatique d'un mode ventilatoire donné parmi plusieurs modes ventilatoires mémorisés, et on commande l'appareil d'assistance respiratoire en appliquant le mode ventilatoire ainsi sélectionné.

Le ventilateur 1 comporte donc des moyens aptes à et conçus pour adapter automatiquement, c'est-à-dire sans intervention humaine, les paramètres de la ventilation mécanique délivrée au patient 20, si les moyens de traitement de signal 8 détectent ou non l'alternance de compression et de relâchement de la cage thoracique, c'est-à-dire l'existence ou non d'un massage cardiaque, dans le but d'assurer une ventilation optimale du patient 20.

Dit autrement, on opère donc un basculement automatique d'un mode ventilatoire donné à un autre mode ventilatoire donné en fonction d'une détection ou détermination de l'existence ou de l'absence de contractions thoraciques, lesdits modes ventilatoires étant mémorisés dans des moyens de mémorisation, telle une mémoire 12.

En particulier, on peut mémoriser plusieurs modes ventilatoires comprenant un premier mode ventilatoire et un second mode ventilatoire qui sont mis en œuvre selon qu'un massage cardiaque ait lieu ou non.

Afin de simplifier les explications, il est considéré ci-après que la pression basse PB est maintenue constante quel que soit le mode de ventilation choisi, c'est-à-dire que PB1=PB2=PB.

Toutefois, comme expliqué ci-avant, la pression basse pourrait aussi varier entre une première pression basse PB1 et une deuxième pression basse PB2, avec PB2>PB1. D'une façon générale, on a donc : PB2 ≥ PB1.

Ainsi, le premier mode ventilatoire qui comprend des valeurs de première pression basse PB1, avec ici PB1=PB, de première pression haute (PH1), avec PH1>PB, de première fréquence (F1) et/ou de première concentration en oxygène (FiO₂-1)) données, est mis en œuvre en cas de détection de massage thoracique

A l'inverse, le second mode ventilatoire qui comprend des valeurs de deuxième pression basse (PB2), avec ici PB2=PB1=PB comme mentionné ci-avant, de deuxième pression haute (PH2), avec PH2>PB et PH2>PH1, de deuxième fréquence (F2), et/ou de deuxième concentration en oxygène (FiO₂-2) données, est mis en œuvre en cas de détection d'absence de massage thoracique ou après arrêt de massage thoracique, c'est-à-dire en cas d'absence ou d'arrêt de contractions thoraciques, par exemple si le cœur du patient de remet à battre « normalement ».

En fait, le ventilateur 1 opère alors un basculement automatique du premier mode ventilatoire vers le deuxième mode ventilatoire de manière à modifier la pression haute, la fréquence et/ou à la concentration en oxygène. Ainsi, si l'on détecte au moins une information relative à une présence ou reprise des contractions thoraciques, l'on opère alors un basculement immédiat du second mode ventilatoire vers le premier mode ventilatoire de manière à modifier la pression haute, la fréquence et/ou à la concentration en oxygène.

Ainsi, selon un mode de ventilation dit « barométrique », on peut opérer une régulation alternée de pression entre plusieurs niveaux de pression comprenant un niveau de pression basse (PB) et plusieurs niveaux de pression haute (PH1, PH2), avec PH2>PH1>PB, la première pression haute (PH1) étant mise en œuvre en cas de détection de massage cardiaque, et la deuxième pression haute (PH2) étant mise en œuvre en cas de non-détection, c'est-à-dire d'absence ou d'arrêt de massage cardiaque. Par exemple, une pression basse (PB) est de l'ordre de 5 cm d'H₂O, la première pression haute (PH1) est de l'ordre de 15 cm d'H₂O et la deuxième pression haute (PH2) est de l'ordre de 20 cm d'H₂O.

De même, la fréquence mise en œuvre peut être augmentée en cas de détection d'une absence ou d'un arrêt du massage cardiaque de manière à compenser la perte de ventilation provoquée par l'arrêt des compressions thoraciques, par exemple la fréquence de ventilation peut augmenter d'une fréquence initiale F1 de l'ordre de 10 cycles/min à une fréquence supérieure F2 de l'ordre de 15 cycles/min. A l'inverse, la fréquence peut repasser de F2 à F1 en cas de redémarrage des compressions thoraciques en cas de nouvel arrêt cardiaque.

Par analogie, on peut également diminuer la FiO2 en cas de détection d'une absence ou d'un arrêt du massage cardiaque, par exemple la FiO2 délivrée peut être de 50%. A l'inverse la FiO2 peut être augmentée en cas de reprise des compressions thoraciques en cas de nouvel arrêt cardiaque, elle peut par exemple passer de 50% à 100%.

De façon alternative ou complémentaire, le ventilateur 1 permet d'adapter automatiquement les paramètres de la ventilation mécanique délivrée au patient 20 pour maintenir une ventilation minute totale constante, à partir des mesures effectuées par les moyens de traitement de signal 8.

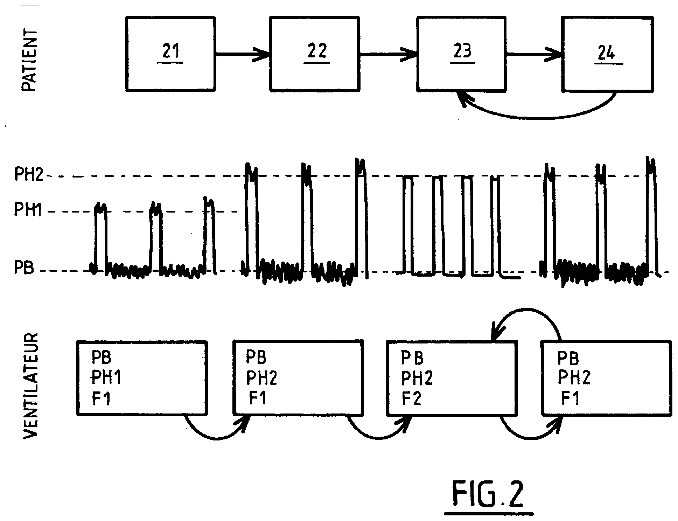

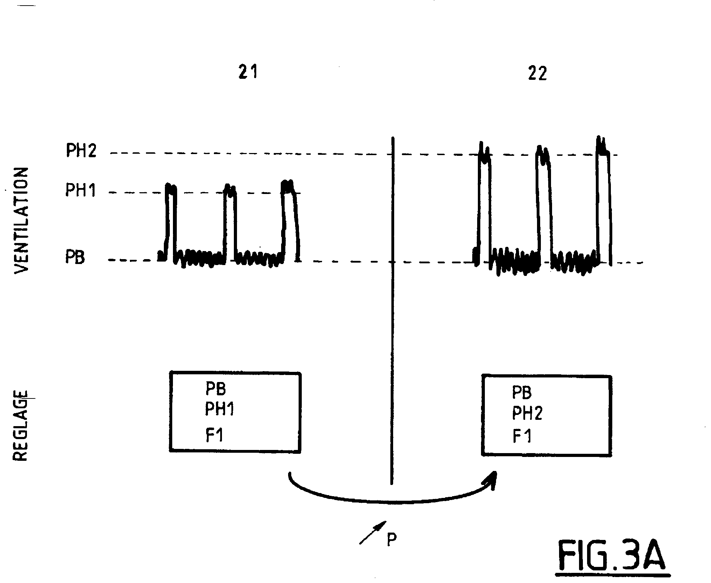

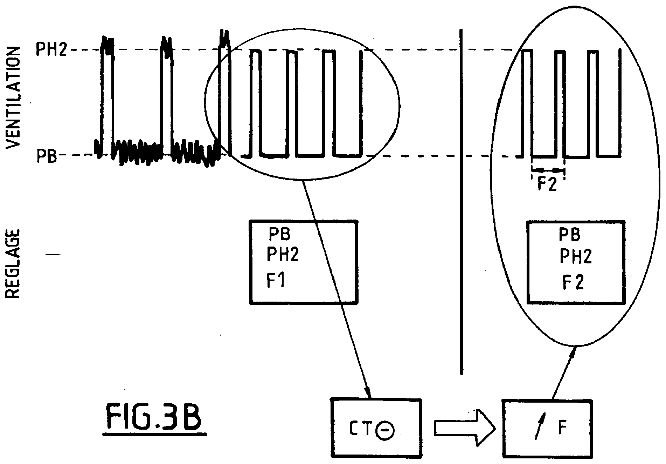

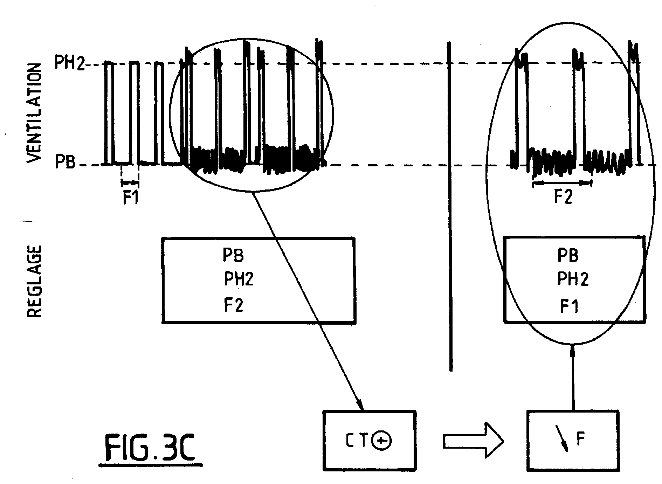

Les paramètres adaptés peuvent être dans le cadre d'une ventilation barométrique entre plusieurs niveaux de pression, les pressions délivrées, en particulier la pression régulée pendant le niveau bas de ventilation, appelée pression basse (PB), et la ou les pressions hautes (PH1, PH2...) régulée(s) pendant le niveau haut, ou encore la fréquence (F) de ventilation, la durée de maintien de la pression basse, la durée de maintien de la pression haute, la pente de montée en pression, le volume délivré à l'insufflation, la nature du gaz délivré...L'adaptation des niveaux de pression est illustrée sur les Figures 2 et 3.

De façon alternative ou complémentaire, le ventilateur 1 peut comporter des moyens de changement des algorithmes d'alarmes, par exemple d'alarmes visant à surveiller le rythme respiratoire du patient, la pression délivrée par le respirateur ou le volume délivré par le respirateur, aptes à et conçus pour passer d'un premier algorithme à un second algorithme, en réponse à une détection par les moyens de traitement de signal, de la réalisation d'un massage cardiaque sur le patient, afin de ne pas alarmer à tort, ce qui perturberait inutilement le secouriste.

Le ventilateur 1 et ses composants requérant de l'énergie pour fonctionner sont alimentés, directement ou indirectement, en courant électrique issu d'une ou plusieurs batteries, rechargeables ou non, de l'alimentation électrique du véhicule de secours qu'il équipe ou du secteur électrique, donc à une tension pouvant aller jusqu'à environ 230 V. Si besoin est, il peut intégrer un convertisseur de courant conçu pour abaisser la tension d'alimentation à une tension d'utilisation de valeur inférieure.

Enfin, une interface homme-machine ou IHM 7, tel un écran d'affichage et de visualisation, tel un écran tactile, permet d'afficher et donc à l'utilisateur de visualiser des informations relatives à la ventilation délivrée, tel que la pression et le débit mesurés par les moyens de mesure 6.

Sont également prévus, des moyens de réglage et de sélection 11, par exemple des boutons poussoirs ou rotatifs, des curseurs, des touches d'activation ou de sélection, ou analogue permettant au personnel soignant d'indiquer au ventilateur 1, la réalisation d'un massage cardiaque et/ou de confirmer au ventilateur 1, la détection qui a été faite de la réalisation d'un massage cardiaque, d'indiquer au ventilateur le type d'interface avec le patient, par exemple masque, sonde d'intubation...

Ces moyens de réglage et de sélection 11 permettent également de modifier, si besoin est, les paramètres de la ventilation mécanique proposés automatiquement par le ventilateur 1, voire même, selon le mode de réalisation considéré, de pouvoir indiquer au ventilateur 1 un changement de la nature du gaz mis en œuvre, par exemple le passage d'air à un mélange air/oxygène ou un changement de teneur en oxygène d'un mélange air/oxygène.

Comme on le voit sur la Figure 1, au moins une partie du circuit de gaz 2, 16, les moyens de traitement de signal 8 et la source de gaz 4 sont agencés dans un capotage ou une carcasse rigide 9 qui forme l'enveloppe externe de l'appareil 1. Cette carcasse 9 inclut ou porte en outre d'autres composants, tel que l'IHM 7, la (ou les) mémoire 12, les moyens de réglage et sélection 11...

La branche inspiratoire 2 du circuit de gaz 2, 16 comprend deux portions distinctes, à savoir une portion interne 2a agencée dans la carcasse rigide 9, par exemple un conduit de gaz, et une portion externe 2b située hors de la carcasse rigide 9 incluant par exemple un tuyau flexible. La portion interne 2a de la branche inspiratoire 2 est en communication fluidique avec la source de gaz 4, à savoir la micro-soufflante motorisée 40 de la Figure 1A ayant une prise ou entrée d'air 4a communiquant avec l'atmosphère ambiante ou la première vanne 41 pilotée de la Figure 1B, de manière à alimenter ladite portion interne 2a avec de l'air, éventuellement enrichi en oxygène.

La micro-soufflante motorisée 40 (Fig. 1A) ou la première vanne 41 pilotée (Fig. 1B) est pilotée par des moyens de pilotage 5, de préférence une carte électronique à microprocesseur, tel un microcontrôleur, mettant en œuvre un (ou des) algorithme(s). De préférence, les moyens de pilotage 5 incluent les moyens de traitement de signal 8, et sont configurés pour piloter la micro-soufflante motorisée 40 ou la première vanne 41 pilotée en fonction des signaux transmis par les moyens de traitement de signal 8.

Par ailleurs, la portion externe 2b de la branche inspiratoire 2 du circuit de gaz 2, 16 située hors de la carcasse rigide 9 est, quant à elle, en communication fluidique avec, du côté amont, la portion interne 2a de la branche inspiratoire 2 et, du côté aval, avec l'interface respiratoire 3, tel un masque ou une sonde d'intubation, de manière à assurer une continuité fluidique entre la source de gaz 4 et le patient 20, et permettre d'acheminer le gaz respiratoire, e.g. l'air provenant de la turbine, jusqu'aux voies aériennes dudit patient.

Sur les Figures 1A et 1B, les moyens de mesure 6, typiquement un (ou plusieurs) capteur, sont agencés sur la portion externe 2b de la branche inspiratoire 2 située hors de la carcasse rigide 9 de manière à opérer les mesures désirées, par exemple de pression et/ou de débit, au sein de ladite portion externe 2b. Bien entendu, les moyens de mesure 6 peuvent être aussi agencés à l'intérieur de la carcasse 9. Dans tous les cas, la liaison entre les moyens de mesure 6 et les moyens de traitement 8 et/ou les moyens de pilotage 5, et donc le transfert des signaux de mesure, se fait au moyen de liaisons filaires par exemple.

Optionnellement, la carcasse 9 peut également comprendre au moins une poignée de portage 13 pour faciliter le transport de l'appareil 1 par l'utilisateur, ce qui est indispensable dans certaines situations d'urgence, et/ou un dispositif d'accrochage 14 permettant l'accroche de l'appareil 1 de ventilation à un support, par exemple une tringle au sein d'un véhicule d'urgence ou un barreau de lit ou de brancard.

Les Figures 2 et 3A à 3C sont des représentations schématiques des différentes étapes de la procédure de prise en charge d'un patient et des adaptations faites par l'appareil d'assistance ventilatoire ou ventilateur 1 selon la présente invention, en particulier la régulation des niveaux de pression du gaz délivré.

Plus précisément, cette procédure comprend les phases successives suivantes :
- Phase 21 de prise en charge (voir Fig. 2 et 3A): le patient en arrêt cardiaque est ventilé au masque et un massage cardiaque commence à lui être appliqué par le personnel soignant. Le ventilateur 1 opère alors une ventilation mécanique au patient en mode barométrique, c'est-à-dire entre plusieurs niveaux de pression, à savoir une valeur de pression initiale, dite « pression basse » ou PB, par exemple 5 cm d'eau, et une première valeur de pression haute (PH1) encore appelée «pression intermédiaire », par exemple 10 cm d'eau, avec PB<PH1, et ce, à une fréquence F1 de ventilation donnée, à savoir ici de 10 cycles/min.
- Phase 22 d'intubation du patient 20 (voir Fig. 2 et 3A): le personnel soignant indique alors au ventilateur 1, un changement d'interface respiratoire, tel que le passage d'une ventilation au masque à une ventilation par sonde d'intubation, en activant un bouton-poussoir ou analogue. Les massages cardiaques sont alors poursuivis par le personnel soignant. Le ventilateur 1 effectue alors automatiquement une adaptation des paramètres de ventilation en augmentant la pression haute de ventilation en la faisant passer de la première pression haute PH1 à une pression supérieure, à savoir la deuxième pression haute PH2, avec : PH2 > PH1. Par exemple, PH2 est égale à 15 cm d'eau. La ventilation est alors poursuivie entre les niveaux PB et PH2. La fréquence F1 est maintenue constante, i.e. à une fréquence F1 de 10 cycles/min.
- Phase 23 de reprise d'une activité cardiaque (voir Fig. 2 et 3B): si une activité cardiaque spontanée est détectée par le personnel soignant, le massage cardiaque est arrêté. Le ventilateur 1 détecte alors l'arrêt du massage cardiaque et effectue automatiquement une adaptation des paramètres de ventilation en augmentant la fréquence de la ventilation mécanique pour compenser la perte de ventilation provoquée par l'arrêt des compressions thoraciques (CT (-) sur Fig. 3B), par exemple la fréquence de ventilation augmente à une fréquence supérieure F2, avec F2>F1, de l'ordre de 15 cycles/min. Les niveaux de pression sont inchangées, la ventilation est poursuivie entre les niveaux PB et PH2.
- Phase 24 en cas de nouvel arrêt cardiaque (voir Fig. 2 et 3C): Après la reprise d'activité cardiaque spontanée en Phase 23, un nouvel arrêt cardiaque peut survenir et nécessite alors une reprise du massage cardiaque par le personnel soignant et une réapparition des contractions thoraciques (CT (+) sur Fig. 3C). Le ventilateur 1 détecte alors cette reprise du massage cardiaque et effectue, là encore, automatiquement une adaptation des paramètres ventilatoires en réduisant la fréquence de la ventilation mécanique, par exemple en la diminuant à son niveau initial F1 de 10 cycles/min, tout en maintenant les niveaux de pression PB et PH2, comme en Phase 23.

Avant d'obtenir une reprise d'activité spontanée pérenne, plusieurs arrêts cardiaques peuvent survenir. Dans ce cas, on procède alors à une alternance entre Phase 23 sans massage cardiaque et Phase 24 avec reprise du massage cardiaque (voir Fig. 2 et 3C). Le ventilateur 1 adapte automatiquement la ventilation mécanique apportée au patient en réduisant la fréquence de ventilation de F2 à F1, à chaque transition de la Phase 23 vers la Phase 24 ou, à l'inverse, en augmentant la fréquence de ventilation de F1 à F2 pour compenser la perte de ventilation provoquée par l'arrêt des compressions thoraciques, à chaque transition de la Phase 24 vers la Phase 23.

Lors des transitions entre les phases 22, 23 et 24, la fréquence de ventilation est adaptée pour délivrer une ventilation suffisante tout comme pourraient l'être les paramètres PB, PH1, PH2, ou encore les concentrations en oxygène délivré appelées FiO2-1 et FiO2-2.

L'appareil d'assistance respiratoire selon la présente invention est utilisable dans le cadre d'une ventilation d'une personne en arrêt cardiaque soumise à des massages cardiaques.

## Revendications

1. Appareil d'assistance respiratoire (1) comprenant
- une micro-soufflante (40) en communication fluidique avec un circuit de gaz (2, 16),
- des moyens de pilotage (5) configurés pour commander ladite micro-soufflante (40),
- des moyens de mesure (6) agencés pour mesurer au moins un paramètre représentatif du flux de gaz acheminé par ledit circuit de gaz (2, 16) et configurés pour convertir ledit au moins un paramètre représentatif dudit flux de gaz en au moins un signal représentatif dudit flux de gaz,
- des moyens de traitement de signal (8) aptes à et conçus pour traiter ledit au moins un signal représentatif du flux de gaz fourni par les moyens de mesure (6), et pour en déduire, au moins une information relative à la réalisation ou à la non-réalisation d'un massage cardiaque sur un patient, et
- une interface homme-machine (IHM) (7),
**caractérisé en ce qu'**il comprend en outre :
- des moyens de mémorisation (12) de modes ventilatoires configurés pour mémoriser plusieurs modes ventilatoires comprenant au moins :
i) un premier mode ventilatoire correspondant à une réalisation de massage cardiaque comprenant des valeurs de première pression basse (PB1) et de première pression haute (PH1), avec PH1>PB1, et
ii) un deuxième mode ventilatoire correspondant à une non-réalisation ou un arrêt de massage cardiaque, comprenant des valeurs de deuxième pression basse (PB2) et de deuxième pression haute (PH2), avec PH2>PB2 et PH2>PH1, et
- des moyens de sélection de mode ventilatoire permettant de sélectionner et appliquer le premier ou le deuxième mode ventilatoire mémorisé, par activation par un utilisateur de moyens de réglage et de sélection (11), en réponse à une détection d'au moins une information relative à une présence de contractions thoraciques ou, inversement, une absence ou un arrêt de contractions thoraciques.

2. Appareil selon la revendication 1, **caractérisé en ce que** :
- les moyens de mémorisation (12) de modes ventilatoires sont configurés pour mémoriser:
i) un premier mode ventilatoire comprenant des valeurs de première pression basse (PB1), de première pression haute (PH1), avec PH1>PB1 et de première fréquence (F1) données, et
ii) un second mode ventilatoire comprenant des valeurs de deuxième pression basse (PB2), de deuxième pression haute (PH2), avec PH2>PB2 et PH2>PH1, et de deuxième fréquence (F2) données, avec F2>F1, et
- les moyens de sélection de mode ventilatoire sont conçus pour opérer un basculement du premier mode ventilatoire vers le deuxième mode ventilatoire, ou inversement, de manière à modifier la pression haute (PH1, PH2) et la fréquence (F1, F2) en réponse à une détection d'au moins une information relative à une absence ou un arrêt de contractions thoraciques ou, inversement, une présence de contractions thoraciques.

3. Appareil selon l'une des revendications précédentes, **caractérisé en ce que** les moyens de réglage et de sélection (11) comprennent une touche située sur un écran tactile de l'interface homme-machine (IHM) (7).

4. Appareil selon l'une des revendications précédentes, **caractérisé en ce qu'**il est conçu pour proposer le premier ou le deuxième mode ventilatoire à l'utilisateur, l'utilisateur pouvant valider le premier ou le deuxième mode ventilatoire proposé par action sur une touche des moyens de réglage et de sélection (11).

5. Appareil selon l'une des revendications précédentes, **caractérisé en ce que** les moyens de mesure (6) comprennent au moins un capteur et/ou les moyens de traitement de signal (8) comprennent au moins une carte électronique, de préférence un contrôleur mettant en œuvre un ou plusieurs algorithmes.

6. Appareil selon l'une des revendications précédentes, **caractérisé en ce que** les moyens de réglage ou de sélection (11) sont actionnables par l'utilisateur pour indiquer à l'appareil (1), la réalisation d'un massage cardiaque, pour confirmer une détection d'un massage cardiaque, pour indiquer le type d'interface respiratoire utilisée ou pour modifier l'un ou des paramètres de ventilation mécanique proposés automatiquement par l'appareil.

7. Appareil selon l'une des revendications précédentes, **caractérisé en ce que** les moyens de mémorisation (12) de modes ventilatoires comprennent au moins une mémoire de stockage de données, en particulier une mémoire flash.

8. Appareil selon l'une des revendications précédentes, **caractérisé en ce que** l'IHM (7) comprend un écran de visualisation d'informations.

9. Appareil selon la revendication 8, **caractérisé en ce que** l'écran de visualisation d'informations (7) est digital et tactile.

10. Appareil selon l'une des revendications précédentes, **caractérisé en ce que** la micro-soufflante (40) est en communication fluidique avec une branche inspiratoire (2) du circuit de gaz (2, 16).

11. Appareil selon l'une des revendications 1 ou 2, **caractérisé en ce que** les moyens de mémorisation (12) de modes ventilatoires sont configurés pour mémoriser un premier mode ventilatoire comprenant des valeurs de première pression basse (PB1) comprise entre 0 et 20 cm d'eau, et de première pression haute (PH1) comprise entre 10 et 60 cm d'eau, avec PH1>PB1, et éventuellement de première fréquence (F1) comprise entre 5 et 40 c/min.

12. Appareil selon l'une des revendications 1 ou 2, **caractérisé en ce que** les moyens de mémorisation (12) de modes ventilatoires sont configurés pour mémoriser un second mode ventilatoire comprenant des valeurs de deuxième pression basse (PB2) comprise entre 0 et 20 cm d'eau, et de deuxième pression haute (PH2) comprise entre 10 et 60 cm d'eau, avec PH2>PB2, PB2>PB1 et PH2>PH1, et éventuellement de deuxième fréquence (F2) comprise entre 5 et 40 c/min, avec F2>F1.

13. Appareil selon la revendication 1, **caractérisé en ce que** la pression basse (PB) est constante avec PB1 = PB2 = PB.

## Patentansprüche

1. Vorrichtung zur Atmungsunterstützung (1), umfassend
- ein Mikrogebläse (40) in fluidischer Kommunikation mit einem Gaskreislauf (2, 16),
- Steuerungsmittel (5), die konfiguriert sind, um das Mikrogebläse (40) zu steuern,
- Messmittel (6), die angeordnet sind, um zumindest einen repräsentativen Parameter der durch den Gaskreislauf (2, 16) zugeführten Gasströmung zu messen, und konfiguriert sind, um den zumindest einen repräsentativen Parameter der Gasströmung in zumindest ein repräsentatives Signal der Gasströmung umzuwandeln,
- Signalbehandlungsmittel (8), die fähig und konzipiert sind, um das zumindest eine repräsentative Signal der Gasströmung, das durch die Messmittel (6) geliefert wird, zu behandeln, und um davon zumindest eine Information in Bezug auf das Ausführen oder auf das Nicht-Ausführen einer Herzmassage an einem Patienten abzuleiten,
- eine Mensch-Maschinen-Schnittstelle (MMS) (7)
**dadurch gekennzeichnet, dass** sie weiter umfasst:
- Speichermittel (12) von Beatmungsmodi, die konfiguriert sind, um mehrere Beatmungsmodi zu speichern, die zumindest Folgendes umfassen:
i) einen ersten Beatmungsmodus, der einem Ausführen einer Herzmassage entspricht, der Werte eines ersten niedrigen Drucks (ND1) und eines ersten hohen Drucks (HD1) umfasst, mit HD1 > ND1, und
ii) einen zweiten Beatmungsmodus, der einem Nicht-Ausführen oder einer Unterbrechung einer Herzmassage entspricht, der Werte eines zweiten niedrigen Drucks (ND2) und eines zweiten hohen Drucks (HD2) umfasst, mit HD2 > ND2 und HD2 > HD1, und
- Mittel zur Auswahl eines Beatmungsmodus, die es ermöglichen, den ersten oder den zweiten gespeicherten Beatmungsmodus durch Aktivieren durch einen Benutzer von Einstell- und Auswahlmitteln (11) als Antwort auf einen Nachweis zumindest einer Information in Bezug auf eine Anwesenheit von Brustkontraktionen oder, umgekehrt, eine Abwesenheit oder eine Unterbrechung von Brustkontraktionen auszuwählen und anzuwenden.

2. Vorrichtung nach Anspruch 1, **dadurch gekennzeichnet, dass**
- die Speichermittel (12) von Beatmungsmodi konfiguriert sind, um Folgendes zu speichern:
i) einen ersten Beatmungsmodus, der gegebene Werte eines ersten niedrigen Drucks (ND1), eines ersten hohen Drucks (HD1), mit HD1>ND1 und einer ersten Frequenz (F1) umfasst, und
ii) einen zweiten Beatmungsmodus, der gegebene Werte eines zweiten niedrigen Drucks (ND2), eines zweiten hohen Drucks (HD2), mit HD2 > ND2 und HD2 > HD1, und einer zweiten Frequenz (F2), mit F2 > F1, umfasst, und
die Mittel zur Auswahl eines Beatmungsmodus konzipiert sind, um ein Umschalten des ersten Beatmungsmodus auf den zweiten Beatmungsmodus, oder umgekehrt, zu betreiben, um den hohen Druck (HD1, HD2) und die Frequenz (F1, F2) als Antwort auf eine Erfassung von zumindest einer Information in Bezug auf eine Abwesenheit oder eine Unterbrechung von Brustkontraktionen oder, umgekehrt, einer Anwesenheit von Brustkontraktionen zu verändern.

3. Vorrichtung nach einem der vorstehenden Ansprüche, **dadurch gekennzeichnet, dass** die Einstell- und Auswahlmittel (11) eine Taste umfassen, die sich auf einem taktilen Bildschirm der Mensch-Maschinen-Schnittstelle (MMS) (7) befindet.

4. Vorrichtung nach einem der vorstehenden Ansprüche, **dadurch gekennzeichnet, dass** sie entworfen ist, um dem Benutzer den ersten oder den zweiten Beatmungsmodus vorzuschlagen, wobei der Benutzer den vorgeschlagenen ersten oder zweiten Beatmungsmodus durch Betätigung auf einer Taste der Einstell- und Auswahlmittel (11) validieren kann.

5. Vorrichtung nach einem der vorstehenden Ansprüche, **dadurch gekennzeichnet, dass** die Messmittel (6) zumindest einen Sensor umfassen und/oder die Signalbehandlungsmittel (8) zumindest eine Elektronikkarte, vorzugsweise ein Steuergerät, das ein oder mehrere Algorithmen implementiert, umfassen.

6. Vorrichtung nach einem der vorstehenden Ansprüche, **dadurch gekennzeichnet, dass** die Einstell- oder Auswahlmittel (11) durch den Benutzer betätigt werden können, um die Vorrichtung (1) auf das Ausführen einer Herzmassage hinzuweisen, um eine Erfassung einer Herzmassage zu bestätigen, um auf den Typ der verwendeten respiratorischen Schnittstelle hinzuweisen oder um einen oder mehrere Parameter mechanischer Beatmung, die automatisch von der Vorrichtung vorgeschlagen werden, zu verändern.

7. Vorrichtung nach einem der vorstehenden Ansprüche, **dadurch gekennzeichnet, dass** die Speichermittel (12) von Beatmungsmodi zumindest einen Datenspeicher, insbesondere einen Flash-Speicher, umfassen.

8. Vorrichtung nach einem der vorstehenden Ansprüche, **dadurch gekennzeichnet, dass** die MMS (7) einen Anzeigebildschirm von Informationen umfasst.

9. Vorrichtung nach Anspruch 8, **dadurch gekennzeichnet, dass** der Anzeigebildschirm (7) von Informationen digital und taktil ist.

10. Vorrichtung nach einem der vorstehenden Ansprüche, **dadurch gekennzeichnet, dass** das Mikrogebläse (40) in fluidischer Kommunikation mit dem Einatmungszweig (2) des Gaskreislaufs (2, 16) steht.

11. Vorrichtung nach einem der Ansprüche 1 oder 2, **dadurch gekennzeichnet, dass** die Speichermittel (12) von Beatmungsmodi konfiguriert sind, um einen ersten Beatmungsmodus zu speichern, der Werte eines ersten niedrigen Drucks (ND1), der zwischen 0 und 20 cm Wasser liegt, und eines ersten hohen Drucks (HD1), der zwischen 10 und 60 cm Wasser liegt, mit HD1 > ND1, und eventuell einer ersten Frequenz (F1), die zwischen 5 und 40 c/min liegt, umfasst.

12. Vorrichtung nach einem der Ansprüche 1 oder 2, **dadurch gekennzeichnet, dass** die Speichermittel (12) von Beatmungsmodi konfiguriert sind, um einen zweiten Beatmungsmodus zu speichern, der Werte eines zweiten niedrigen Drucks (ND2), der zwischen 0 und 20 cm Wasser liegt, und eines zweiten hohen Drucks (HD2), der zwischen 10 und 60 cm Wasser liegt, mit HD2 > ND2, ND2 ≥ ND1 und HD2 > HD1, und eventuell einer zweiten Frequenz (F2), die zwischen 5 und 40 c/min liegt, mit F2 > F1, umfasst.

13. Vorrichtung nach Anspruch 1, **dadurch gekennzeichnet, dass** der niedrige Druck (ND) konstant mit ND1 = ND2 = ND ist.

## Claims

1. Respiratory assistance device (1) comprising
- a micro-fan (40) communicating fluidically with a gas circuit (2, 16),
- control means (5) configured to control said micro-fan (40),
- measurement means (6) arranged to measure at least one parameter representative of the gas flow transported by said gas circuit (2, 16) and configured to convert said at least one parameter representative of said gas flow into at least one signal representative of said gas flow,
- signal processing means (8) capable of and designed to process said at least one signal representative of the gas flow provided by the measurement means (6), and to deduct from this, at least one item of information relating to the carrying out or the non-carrying out of a cardiac massage on a patient, and
- a man-machine interface (MMI) (7),
**characterised in that** it further comprises:
- ventilatory mode memorisation means (12) configured to memorise several ventilatory modes comprising at least:
i) one first ventilatory mode corresponding to a carrying out of a cardiac massage comprising values of first low pressure (PB1), of first high pressure (PH1), with PH1>PB1, and
ii) one second ventilatory mode corresponding to a non-carrying out or a stopping of a cardiac massage, comprising values of second low pressure (PB2) and of second high pressure (PH2), with PH2>PB2 and PH2>PH1, and
- ventilatory mode selection means making it possible to select and apply the first or the second memorised ventilatory mode, by activation by a user of adjustment and selection means (11), in response to a detection of at least one item of information relating to a presence of thoracic contractions or, conversely, an absence or a stopping of thoracic contractions.

2. Device according to claim 1, **characterised in that**:
- the ventilatory mode memorisation means (12) are configured to memorise:
i) a first ventilatory mode comprising given values of low pressure (PB1), of first high pressure (PH1), with PH1>PB1 and of first frequency (F1), and
ii) a second ventilatory mode comprising given values of second low pressure (PB2), of second high pressure (PH2), with PH2>PB2 and PH2>PH1, and of second frequency (F2), with F2>F1, and
- the ventilatory mode selection means are designed to actuate a changeover of the first ventilatory mode to the second ventilatory mode, or conversely, so as to modify the high pressure (PH1, PH2) and the frequency (F1, F2) in response to a detection of at least one item of information relating to an absence of a stopping of thoracic contractions or, conversely, a presence of thoracic contractions.

3. Device according to one of the preceding claims, **characterised in that** the adjustment and selection means (11) comprise a key located on a touchscreen of the man-machine interface (MMI) (7).

4. Device according to one of the preceding claims, **characterised in that** it is designed to propose the first or the second ventilatory mode to the user, the user able to confirm the first or the second ventilatory mode proposed by action on a key of the adjustment and selection means (11).

5. Device according to one of the preceding claims, **characterised in that** the measurement means (6) comprise at least one sensor and/or the signal processing means (8) comprise at least one electronic board, preferably a controller implementing one or more algorithms.

6. Device according to one of the preceding claims, **characterised in that** the adjustment or selection means (11) are actuatable by a user to indicate to a device (1), the carrying out of a cardiac massage, to confirm a detection of a cardiac massage, to indicate the type of respiratory interface used or to modify one or more mechanical ventilation parameters proposed automatically by the device.

7. Device according to one of the preceding claims, **characterised in that** the ventilatory mode memorisation means (12) comprise at least one data storage memory, in particular a flash memory.

8. Device according to one of the preceding claims, **characterised in that** the MMI (7) comprises a screen for viewing information.

9. Device according to claim 8, **characterised in that** the screen for viewing information (7) is digital and touch-sensitive.

10. Device according to one of the preceding claims, **characterised in that** the micro-fan (40) communicates fluidically with an inspiratory branch (2) of the gas circuit (2, 16).

11. Device according to one of claims 1 or 2, **characterised in that** the ventilatory mode memorisation means (12) are configured to memorise a first ventilatory mode comprising values of low pressure (PB1) of between 0 and 20 cm of water, of first high pressure (PH1) of between 10 and 60 cm of water, with PH1>PB1, and optionally of first frequency (F1) of between 5 and 40 c/min.

12. Device according to one of claims 1 or 2, **characterised in that** the ventilatory mode memorisation means (12) are configured to memorise a second ventilatory mode comprising values of second low pressure (PB2) of between 0 and 20 cm of water, of second high pressure (PH2) of between 10 and 60 cm of water, with PH2>PB2, PB2>PB1 and PH2>PH1, and optionally of second frequency (F2) of between 5 and 40 c/min, with F2>F1.

13. Device according to claim 1, **characterised in that** the low pressure (PB) is constant with PB1 = PB2 = PB.
